# EUROPEAN PATENT SPECIFICATION

(11) **EP 1 200 059 B1**
(45) Date of publication and mention of the grant of the patent: **01.10.2003**
(21) Application number: 00949315.6
(22) Date of filing: 11.07.2000
(51) Int. Cl.: A61K 7/48

(54) **SKIN CARE COMPOSITION**
HAUTPFLEGEZUSAMMENSETZUNG
COMPOSITION DE SOIN DE LA PEAU

(30) Priority: 30.07.1999 GB 9918025
(43) Date of publication of application: 02.05.2002
(73) Proprietor: UNILEVER PLC, London EC4P 4BQ (GB); UNILEVER N.V., 3013 AL Rotterdam (NL)
(72) Inventor: BARRETT, Karen, Elizabeth, Bedford, Bedfordshire MK44 1LQ (GB); GREEN, Martin, Richard, Bedford, Bedfordshire MK44 1LQ (GB); RAWLINGS, Anthony, V., Unilever Res. Port Sunlight, Wirral, Merseyside CH63 3JW (GB)
(74) Representative: Elliott, Peter William
(86) International application number: EP0006595
(87) International publication number: WO01008650

(56) References cited:
- EP-A- 0 742 005
- EP-A- 0 803 247
- WO-A-98/13020
- WO-A-99/26588
- FR-A- 2 780 886
- US-A- 5 451 405
- US-A- 5 723 139
- US-A- 5 759 556

## Description

### FIELD OF THE INVENTION

This invention relates to topical compositions for application to human skin and to their use in improving the condition and appearance of skin.

### BACKGROUND OF THE INVENTION

Skin is subject to deterioration through dermatological disorders, environmental abuse (wind, air conditioning, and central heating) or through the normal ageing process (chronoageing) which may be accelerated by exposure of skin to sun (photoageing). In recent years the demand for cosmetic compositions and cosmetic methods for improving the appearance and condition of skin has grown enormously.

Consumers are increasingly seeking anti-ageing cosmetic products that treat or delay the visible signs of chronoageing and photoageing skin such as wrinkles, lines, sagging, hyperpigmentation and age spots.

Consumers also frequently seek other benefits from cosmetic products in addition to anti-ageing. The concept of sensitive skin has also raised the consumer demand for cosmetic products that improve the appearance and condition of sensitive, dry and/or flaky skin and to soothe red, and/or irritated skin. Consumers also desire cosmetic products that have an oil/sebum control effect.

Many people are concerned with the degree of pigmentation of their skin. For example, people with age spots or freckles may wish such pigmented spots to be less pronounced. Others may wish to reduce the skin darkening caused by exposure to sunlight or to lighten their natural skin colour. To meet this need many attempts have been made to develop products that reduce the pigment production in the melanocytes. However, the substances thus far identified tend to have undesirable side effects, e.g. skin irritation.

Consequently such substances are not suitable for cosmetic use, or they can only be applied at a concentration at which their skin lightening effect is less than desired. Using a combination of different skin lightening substances may be considered to reduce adverse side effects, but there is a substantial risk that by using such a combination the skin lightening is reduced as well due to competition effects. Therefore there is a need for improvement in the effectiveness of cosmetic skin lightening products particularly, such that they do not irritate the skin.

Skin care cosmetic and dermatological compositions for improving the condition and appearance of skin comprising long chain triglyceride esters of polyunsaturated essential fatty acids, the free acids and their alkali or ammonium salts are well known in the art. For instance, GB 2181349 A describes *inter alia* a composition composed of triglycerides of linoleic acid for improving the smoothness and elasticity of skin. A commercial product, Linola Fett n, ex. Dr. August Wolff Gmbh, is available for the treatment of dry skin diseases, and dermatoses, which contains *inter alia* a mixture of the 9,11 isomers of conjugated linoleic acid.

Retinal (vitamin A) is an endogenous compound that occurs naturally in the human body and is essential for normal epithelial cell differentiation. Natural and synthetic vitamin A derivatives (retinoids) have been used extensively in the treatment of a variety of skin disorders and have been used as skin repair or renewal agents. Retinoic acid, for example, has been employed to treat a variety of skin conditions, e.g., acne, wrinkles, psoriasis, age spots and discoloration. See e.g., Vahlquist, A. et al., J. Invest. Dermatol., Vol. 94, Holland D. B. and Cunliffe, W. J. (1990), pp. 496-498; Ellis, C. N. et al., "Pharmacology of Retinols in Skin", Vasel, Karger, Vol. 3, (1989), pp. 249-252; Lowe, N. J. et al., "Pharmacology of Retinols in Skin", Vol. 3, (1989), pp. 240-248, PCT Patent Application No. WO 93/19743.

WO99/26588 describes cosmetic anti-aging skin creams comprising conjugated linoleic acid and optionally retinoyl ascorbate, which is a retinoic acid ester.

Various other patent applications include descriptions of compositions containing linoleic acid and retinol and/or its derivatives. These include WO-A-98/13020, US-A-5,759,556, US-A-5,723,139, EP-A-742,005, and US-A-5,451,405.

There continues to be a need, however, for alternative effective cosmetic compositions for topical application to skin for treating/delaying the visible signs of ageing and photodamaged skin such as wrinkles, lines, sagging, hyperpigmentation and age spots.

We have now found that effective treatment and prevention of normal (but cosmetically undesirable) skin conditions due to chronoageing or photoageing, such as wrinkles, lines, sagging, hyperpigmentation and age spots, may be obtained through the application of cosmetic compositions to the skin which consist of a specific fatty acid - conjugated linoleic acid and/or derivatives thereof in combination with retinoic acid, retinol or an ester of retinol (a retinyl ester) and/or an inhibitor of the enzyme acyl CoA retinol transferase(ARAT) or the enzyme lecithin retinol acyl transferase (LRAT) (hereinafter referred to as a LRAT/ARAT inhibitors). We have also found that the use of such cosmetic compositions advantageously provides further skin benefits in addition to anti-ageing such as soothing sensitive and/or irritated skin, controlling oil/sebum secretion and for lightening the skin.

The art discussed above does not disclose the specific synergistic combination of conjugated linoleic acid with retinoic acid, retinol or a retinyl ester and/or LRAT/ARAT inhibitors, nor the use of such a specific combination for treating wrinkles, sensitive skin, dry skin, controlling oil/sebum secretion, or lightening skin.

### SUMMARY OF THE INVENTION

According to a first aspect of the present invention there is provided a topical composition comprising:
(a) conjugated linoleic acid and/or derivatives thereof;
(b) retinoic acid, retinol, retinyl ester and/or a LRAT/ARAT inhibitor; and
(c) a dermatologically acceptable vehicle.

According to a second aspect of the present invention there is provided a cosmetic method of providing at least one skin care benefit selected from: treating/preventing wrinkling, sagging, dry, aged and/or photodamaged skin; boosting collagen deposition in skin, boosting decorin production in skin, enhancing tissue repair; soothing irritated, red and/or sensitive skin; improving skin texture, smoothness and/or firmness; lightening skin; controlling oil/sebum secretion, the method comprising applying to the skin a topical composition as described above.

The present invention also encompasses the use of the inventive compositions for providing at least one skin care benefit selected from treating/preventing wrinkling, sagging, aged and/or photodamaged skin; boosting collagen deposition in skin, boosting decorin production in skin, enhancing tissue repair; soothing irritated, red and/or sensitive skin; improving skin texture, smoothness and/or firmness; lightening skin; and controlling oil/sebum secretion.

According to a still further aspect of the present invention there is provided the use of conjugated linoleic acid and derivatives thereof in combination with retinoic acid, retinol, retinyl ester and/or a LRAT/ARAT inhibitor in a cosmetic topical composition for providing at least one cosmetic skin care benefit selected from treating/preventing wrinkling, sagging, aged and/or photodamaged skin; boosting collagen deposition in skin, boosting decorin production in skin, enhancing tissue repair; soothing irritated, red and/or sensitive skin; improving skin texture, smoothness and/or firmness; lightening skin; and controlling oil/sebum secretion.

The inventive compositions, methods and uses thus provide anti-ageing benefits which result in the promotion of smooth and supple skin with improved elasticity and a reduced or delayed appearance of wrinkles and aged skin, with improved skin colour. A general improvement in the appearance, texture and condition, in particular with respect to the radiance, clarity, and general youthful appearance of skin is achieved. The inventive compositions, methods and uses are also beneficial for soothing and calming sensitive and/or irritated skin, for lightening skin and for controlling oil/sebum secretion. Thus the present invention advantageously provides a wide range of skin care benefits.

The term treating as used herein includes within its scope reducing, delaying and/or preventing the above mentioned normal skin conditions such as wrinkled, aged, and/or photodamaged, and/or irritated skin and generally enhancing the quality of skin and improving its appearance and texture by preventing or reducing irritation, wrinkling and increasing flexibility, firmness, smoothness, suppleness and elasticity of the skin. The compositions, methods and uses according to the invention may be useful for treating skin which is already in a wrinkled, aged, photodamaged, irritated condition or for treating youthful skin to prevent or reduce those aforementioned undesirable changes due to the normal ageing/photoageing process.

### DETAILED DESCRIPTION OF THE INVENTION

Conjugated linoleic acid (hereinafter referred to as CLA) is a diunsaturated long chain (C18) fatty acid. CLA comprises a group of positional and geometric isomers of linoleic acid in which various configurations of cis and trans double bonds at positions (6, 8), (7, 9), (8, 10), (9, 11), (10, 12) or (11, 13) are possible. Thus twenty-four different isomers of CLA exist.

The invention also includes derivatives of the free acid which thus comprise conjugated linoleic acid moieties. Preferable derivatives include those derived from substitution of the carboxyl group of the acid, such as esters (e.g. triglyceride esters, monoglyceride esters, diglyceride esters, phosphoesters), amides (e.g. ceramide derivatives), salts (e.g. alkali metal and alkali earth metal salts, ammonium salts); and/or those derived from substitution of the C18 carbon chain, such as alpha hydroxy and/or beta hydroxy derivatives.

In the case of triglyceride ester derivatives, all positional isomers of CLA substituents on the glycerol backbone are included. The triglycerides must contain at least one CLA moiety. For example, of the three esterifiable positions on the glycerol backbone, the 1 and 2 positions may be esterified with CLA and by another lipid at position 3 or as an alternative, the glycerol backbone could be esterified by CLA at the 1 and 3 positions with another lipid at position 2.

The most preferred isomers of CLA for use in the present invention is the cis 9 trans 11 (c9 t11) or trans 10 cis 12 (t10 c12) isomer. Preferably at least 1% by weight of the total CLA and/or CLA moieties present in the composition is in the form of the c9, t11 and/or t10, c12 isomer. More preferably at least 20% and most preferably at least 40%, by weight of the total CLA and/or CLA moieties present in the composition, is in the form of the c9, t11 isomer and/or t10, c12 isomer.

In a particularly preferred embodiment the conjugated linoleic acid is enriched in the c9 t11 or the t10, c12 isomer. By 'enriched' is meant that at least 50% by weight of the total CLA (and/or CLA) moieties present in the composition is in the form of the cis 9, trans 11 or the trans 10 cis 12 isomer. Preferably, at least 70%, more preferably at least 80%, and most preferably at least 90% by weight of the total CLA and/or CLA moieties present in the composition, is in the form of the c9, t11 isomer or the t10 c12 isomer.

The CLA and/or derivatives thereof comprising CLA moieties according to the present invention are commercially available as oils that are rich in conjugated linoleic acid triglyceride such as Tung oil or as dehydrated castor oil (Unichema). A mix isomer product is available from Sigma and a c9 t11 isomer enriched CLA is available from Matreya inc. Alternatively CLA according to the preferred embodiments of the present invention may be prepared according to the method disclosed in WO 97/18320 whose contents are incorporated herein by reference. A preferred method of preparation is disclosed in Example 1 below.

Wherever the term 'conjugated linoleic acid' or 'CLA' is used in this specification it is to be understood that the derivatives thereof comprising CLA moieties are also included. 'CLA moieties' refers to CLA fatty acyl portion(s) of a CLA derivative.

The CLA, to be employed in accordance with the present invention is present in the topical composition in an effective amount. Normally the total amount of the active is present in an amount between 0.0001% and 50% by weight of the composition. More preferably the amount is from 0.01% to 10% and most preferably from 0.1% to 5% in order to maximise benefits at a minimum cost.

The compositions according to the present invention also specifically include retinoic acid, retinol, retinyl ester and/or an LRAT/ARAT inhibitor.

The term "retinol" includes the following isomers of retinol: all-trans-retinol, 13-cis-retinol, 11-cis-retinol, 9-cis-retinol, 3,4-didehydro-retinol. Preferred isomers are all-trans-retinol, 13-cis-retinol, 3,4-didehydro-retinol, 9-cis-retinol. Most preferred is all-trans-retinol, due to its wide commercial availability.

Retinyl ester is an ester of retinol. The term "retinol" has been defined above. Retinyl esters suitable for use in the present invention are C₁-C₃₀ esters of retinol,
preferably C₂-C₂₀ esters, and most preferably C₂-C₃, and C₁₆
esters because they are more commonly available The preferred ester for use in the present invention is selected from retinyl palmitate, retinyl acetate, retinyl propionate and retinyl linoleate, because these are the most commercially available and therefore the cheapest. Retinyl ester is also preferred due to its efficacy.

### LRAT/ARAT Inhibitor

Retinol is an endogenous compound that occurs naturally in the human body and is essential for normal epithelial cell differentiation. Esters of retinol hydrolyse in-vivo to produce retinol. It is believed that retinyl esters and retinol are metabolically converted in the skin into retinoic acid according to the following mechanism

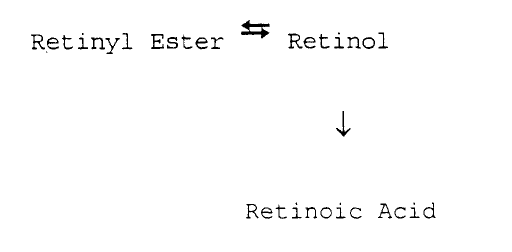

However, most of the endogenously applied retinol is rapidly converted into inactive fatty esters for storage in epidermal cells (keratinocytes).

Esterification of retinol into inactive retinyl esters is achieved in cells by transfer of a fatty acyl group from an acyl CoA, catalysed by the enzyme acyl CoA retinol transferase (ARAT), or by the transfer of an acyl group from phosphatidyl choline, catalysed by the enzyme lecithin retinol acyl transferase (LRAT). These esterification reactions are very efficient in keratinocytes--the majority (95%) of cellular retinoids are in the form of retinyl fatty esters.

The term 'LRAT/ARAT inhibitor' in the present application thus means an agent which inhibits these esterification reactions and thus potentiates the action of retinol by increasing the amount of retinol available for conversion to retinoic acid.

The LRAT/ARAT inhibitors within the scope of the present invention are identifiable as those compounds which at 100 µM concentration inhibit at least 20% of LRAT or ARAT catalysed retinol esterification as measured by the in vitro Microsomal Assay described below in Example 3. In a preferred embodiment of the invention, the LRAT/ARAT inhibitor is a compound that at 100 µM concentration inhibits at least 40% and most preferably at least 50% of LRAT or ARAT catalysed retinol esterification. The in vitro Microsomal Assay employed for determining whether or not a compound is such a LRAT/ARAT inhibitor is as described in Example 3 below.

Thus if a compound passes this in vitro Microsomal assay, that is, it inhibits sufficiently an LRAT or ARAT catalysed retinol esterification as measured by the in vitro Microsomal Assay, it is included in the present invention even if it is not specifically mentioned herein.

Examples of such LRAT/ARAT inhibitors include fatty acid amides, hydroxy fatty acid amides, ceramides, melinamide, imidazolidinones, and cyclic aliphatic unsaturated hydrocarbons, terpenes and fatty hydroxyethyl imidazoline surfactants.

### Cyclic Aliphatic Unsaturated Compounds

Suitable cyclic aliphatic unsaturated compounds are selected according to the in-vitro Microsomal Assay Test described above.

A preferred cyclic aliphatic unsaturated compound is selected from cyclic aliphatic unsaturated aldehydes, ketones, alcohols and esters such as alpha damascone, beta damascone, delta damascone, isodamascone, damascenone, alpha ionone, beta ionone, allyl alpha ionone, isobutyl ionone, alpha methyl jonone, gamma methyl ionone, brahmanol, sandanol, alpha terpineol, lyral, ethyl saffranate, and mixtures thereof. Preferably, in order to maximize performance at a minimum cost, a cyclic aliphatic unsaturated compound is selected from the group consisting of damascones and ionones.

Most preferably, the cyclic aliphatic unsaturated compound is a α-Damascone and/or α-Ionone.

### Diterpenes

Suitable diterpenes are selected according to the in-vitro Microsomal Assay Test described above. A preferred diterpene compound is geranyl geraniol, which is a potent inhibitor of retinol esterification.

### Fatty Hydroxethyl Imidazoline Surfactants

Fatty hydroxyethyl imidazoline surfactants included in the present invention pass the in-vitro Microsomal Assay test described above. Preferred fatty hydroxyethyl imidazolines have the following general structure: 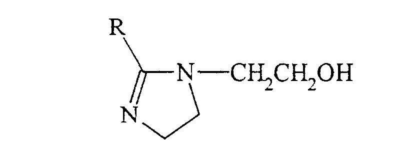 wherein R is an aliphatic saturated or unsaturated, straight or branched hydro-carbon chain containing from 8 to 20 carbon atoms.

Preferably, R in the fatty hydroxyethyl imidazoline contains from 8 to 18 carbon atoms, more preferably from 11 to 18 carbon atoms. Most preferably, the fatty hydroxyethyl imidazoline is oleyl hydroxyethyl imidazoline, due to its commercial availability and efficacy.

### Fatty Acid Amide

Preferably, the fatty acid amide contains at least 6 carbon atoms. Suitable fatty acids include saturated and unsaturated, straight or branched fatty acids. Suitable fatty acids preferably contain from 8 to 24 carbon atoms, preferably from 12 to 20 carbon atoms, and most preferably from 12 to 18 carbon atoms, because longer chain fatty acid amides are more beneficial for conditioning of the skin. In the most preferred embodiment of the invention, amides of essential fatty acids are employed because essential fatty acids provide nutrition for the skin. Examples of essential fatty acids include but are not limited to linoleic, linolenic, arachidonic, gamma-linolenic, homo-gamma-linolenic, and mixtures thereof. Linoleic acid is most preferred because it is also a precursor to ceramide.

The preferred amides included in the present invention are mono- and di-alkanol amides, particularly of essential fatty acids. Alkanol amides are more commonly available than alkyl amides.

The most preferred fatty acid amides are selected from mono-and diethanolamides and phosphatidylethanolamines of linoleic acid, palmitic acid, and coconut oil, diethyl cocamide, linoleamidyl dimethylamine, dimethyl linoleamide, diethyl linoleamide, dimethyl palmitide, myristoyl sarcosine.

### Hydroxy Fatty Acid Amides

The structure of an amide of a hydroxy fatty acid is as follows: 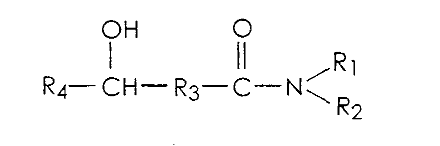 wherein R₁, R₂ and R₄ each is independently selected from hydrogen and aliphatic saturated or unsaturated, straight or branched hydrocarbon chains which may be hydroxylated, containing from 1 to 20 carbon atoms;
R₃ is -(CH₂)ₙ where n is an integer from 0 to 18;

Preferably, R₁, R₂, R₄ each independently contains from 2 to 20 carbon atoms, more preferably from 2 to 15 carbon atoms, most preferably from 3 to 13 carbon atoms.

Preferably the hydroxy acid amide is an amide of α- or β-hydroxy acid, i.e., n is 0 or 1.

The most preferred hydroxy fatty acid amides to be included in the inventive compositions are: lactamide-monoethanolamide, C₁₃-β-hydroxy acid amide (2-hydroxy-C₁₃-amide), N-hydroxyethyl-2-hydroxy-C₁₆ amide, 12-hydroxy-N-(2-hydroxyethyl) octadecanamide, and monoethanolamide of castor oil.

### Polycyclic Triterpene Carboxylic acid (PTCA)

A further example of a suitable LRAT/ARAT inhibitor is a PCTA which passes the in vitro Microsomal Assay.

Preferably the PTCA is a pentacyclic triterpene monocarboxylic acid.

Most preferably, PTCA is selected from the group consisting of ursolic acid, oleanolic acid, glycerrhetinic and glycyrrhizic acid.

PTCA are commercially available from Aldrich and Sigma. Plant extracts containing PTCA are suitable for use in the present invention e.g. *Rosmarinus officinalis* (rosemary), *Diospyros spp*. (persimmon), *Forsythia suspensa* (forsythia), *Lavandula angustifolia* (lavender), *Prunella vulgaris* (selfheal), *Paeonia lactifolia, Glycyrrhiza glabra (licorice)*.

It should be understood that depending on the pH of the composition, PTCA may be present in the composition as a salt, e.g. alkali or alkaline earth salt.

### Ceramides

The ceramides may for example be naturally occurring ceramides, phyto ceramides short chain ceramides, pseudoceramides or neoceramides. The general structure of these molecules is described in EP A 711558 whose contents are hereby incorporated by reference. The most preferred ceramide derivative is acetyl sphingosine due to its efficacy.

The retinoic acid, retinol, retinyl esters and/or LRAT/ARAT inhibitor can be included in the inventive compositions in an amount ranging from 0.0001% to 50% by weight of the composition, preferably from 0.01% to 10%, most preferably from 0.1% to 5%.

### Dermatologically Acceptable Vehicle

The composition used according to the invention also comprises a dermatologically/cosmetically acceptable vehicle to act as a dilutant, dispersant or carrier for the actives. The vehicle may comprise materials commonly employed in skin care products such as water, liquid or solid emollients, silicone oils, emulsifiers, solvents, humectants, thickeners, powders, propellants and the like.

The vehicle will usually form from 5% to 99.9%, preferably from 25% to 80% by weight of the composition, and can, in the absence of other cosmetic adjuncts, form the balance of the composition.

### Optional Skin Benefit Materials and Cosmetic Adjuncts

Besides the actives, other specific skin-benefit actives such as sunscreens, other skin lightening agents, skin tanning agents may also be included. The vehicle may also further include adjuncts such as perfumes, opacifiers, preservatives, colourants and buffers.

### Product Preparation, Form, Use and Packaging

To prepare the topical composition used in the method of the present invention, the usual manner for preparing skin care products may be employed. The active components are generally incorporated in a dermatologically/cosmetically acceptable carrier in conventional manner. The active components can suitably first be dissolved or dispersed in a portion of the water or another solvent or liquid to be incorporated in the composition. The preferred compositions are oil-in-water or water-in-oil or water-in-oil-in-water emulsions.

The composition may be in the form of conventional skin-care products such as a cream, gel or lotion, capsules or the like. The composition can also be in the form of a so-called 'wash-off' product e.g. a bath or shower gel, possibly containing a delivery system for the actives to promote adherence to the skin during rinsing. Most preferably the product is a 'leave-on' product, that is a product to be applied to the skin without a deliberate rinsing step soon after its application to the skin.
The composition may packaged in any suitable manner such as in a jar, a bottle, tube, roll-ball, or the like, in the conventional manner. It is also envisaged that the inventive compositions could be packaged as a kit of two separate compositions one containing the conjugated linoleic acid and the second containing the retinoic acid, retinol, retinyl ester/LRAT/ARAT inhibitor compound, to be applied to the skin simultaneously or consecutively.

The composition according to the present invention may also be formulated in a form suitable for oral ingestion such as a tablet, capsule or similar.

The method of the present invention may be carried out one or more times daily to the skin which requires treatment. The improvement in skin appearance will usually become visible after 3 to 6 months, depending on skin condition, the concentration of the active components used in the inventive method, the amount of composition used and the frequency with which it is applied. In general, a small quantity of the composition, for example from 0.1 to 5 ml is applied to the skin from a suitable container or applicator and spread over and/or rubbed into the skin using the hands or fingers or a suitable device. A rinsing step may optionally follow depending on whether the composition is formulated as a 'leave-on' or a 'rinse-off' product.

In order that the present invention may be more readily understood, the following examples are given, by way of illustration only.

### EXAMPLES

### Example 1

This example illustrates the synthesis of conjugated linoleic acid comprising the c9 t11 isomer and t10 c12 isomer

### Production of Mixed Isomers of CLA

'Analar Reagent' (AR) sodium hydroxide (0.6kg) was dissolved in 6kg of pharmaceutical grade propylene glycol by mixing and heating to 80-85°C. The sample was cooled and 2kg of safflower oil was added. Using standard pilot scale equipment the mixture was refluxed for 3 hours with fast stirring at 170°C. The reaction mix was cooled to about 95°C, the stirrer reduced to an intermediate speed, and the mix neutralised using 1.280 litres of 35.5% hydrochloric acid dissolved in demineralised water (8 litres), keeping the temperature at about 90°C. The reaction mix was allowed to settle and the aqueous phase was run off. The oil phase was washed with 2 x 1 litre of 5% AR salt solution and by 2 x 1 litre of demineralised water at 90°C, discarding any soapy material. The CLA enriched oil was dried at 100°C under vacuum before draining at about 50°C and was filtered through a Buchner system containing a Whatman filter and a thin layer of celite-hyflo-filter aid. The mixed isomer CLA oil was stored under nitrogen at -25°C until required. The composition of the oil produced by this method is set out in table 1 below:

**TABLE 1**

| **Composition of mixed CLA fatty acids (wt%):** | **Relative Percentage of Total fatty acid lipid** |
|---|---|
| c9,t11 | 34.1 **(47% of total CLA)** |
| t10,c12 | 34.1 **(47% of total CLA)** |
| c9,c11 & c10,c12 | 2.3 |
| t9,t11t & t10,12t | 0.7 |
| Other CLA | 1.4 |
| **Total CLA** | **72.6** |
| 16:0 | 7.0 |
| 16:1 | 0.8 |
| 18:0 | 2.5 |
| 18:1 | 13.3 |
| 18:2 (non-CLA) | 3.3 |
| Other fatty acid | 0.5 |

### 2. Production of c9 t11 isomer enriched CLA

### (I) Preparation of lauryl esters:

CLA prepared from Safflower (2.0kg) was added to 2 x molar equivalents of lauryl alcohol (1-dodecanol; 98% ex Aldrich chemicals) along with 5.96kg of demineralised water. The temperature was adjusted to 25°C and 1% (w/w) of *Geotrichum Candidum* (ex Amano Pharmaceuticals, Japan) was added premixed with a little water, and mixed vigorously. The reaction was stopped at 44 hours. The vessel was heated to 80-90°C, the aqueous layer drained and the oil was washed with demineralised water and dried at 100°C under vacuum for 30 minutes. The oil was cooled to 50°C and filtered through a Buchner system containing a Whatman filter and a thin layer of celite-hyflo-filter aid.

### (II) Separation of the enriched c9,t11 CLA esters:

Residual lauryl alcohol was removed at 130°C at 25-35ml per minute by molecular distillation. The residue was coarsely separated into the lauryl esters (enriched in c9,t11 CLA) and free acids (enriched in t10,c12 CLA) by evaporation at 158°C at a flow rate of 25-35ml per minute. Any remaining free acids in the lauryl ester residue were reduced by a further distillation at 171°C at a flow rate of 30-40ml per minute. 2790g of lauryl ester residue was neutralised at 90°C using 330ml of 4M AR sodium hydroxide, followed by separation of the oil from the aqueous phase, 3x washes of the oil in demineralised hot water, a further 0.1M alkali wash and two hot water washes. The enriched lauryl ester oil sample was dried as before.

### (III) Saponification of the enriched c9,t11 CLA lauryl esters:

Lauryl esters of c9,t11 enriched CLA were saponified using AR sodium hydroxide/96% food grade ethanol and re-acidified using AR concentrated hydrochloric acid. The reaction mix containing the enriched CLA free fatty acids was dried at 100°C and filtered as before at about 50°C. Lauryl alcohol was evaporated off at 132°C at 25-30ml per minute. In order to remove any residual lauryl alcohol, free alcohols were esterified to the fatty acids present in the reaction mix, using SP392 *Mucor miehei* lipase (5%, batch lux 0110 ex Novo Nordisk). The enriched C9 t11 CLA containing fatty acids were separated from the lauryl esters using molecular distillation under vacuum at 155°C at 15-20ml per minute. The composition of the enriched C9 t11 CLA produced by the above method is set out in table 2 below:

**TABLE 2**

| **Composition of typical preparation of enriched c9,t11 CLA fatty acids (wt%):** | **Relative Percentage of Total Fatty Acid Lipid** |
|---|---|
| C9,t11 | 66.1 **(93% of total CLA)** |
| T10,c12 | 4.1 |
| C9,c11 & c10,c12 | 0.3 |
| T9,t11t & t10,12t | 0.4 |
| Other CLA | 0.2 |
| **Total CLA** | **71.1** |
| 16:0 | 1.6 |
| 16:1 | - |
| 18:0 | 0.4 |
| 18:1 | 22.3 |
| 18:2 (non-CLA) | 4.5 |
| Other fatty acid | 0.1 |

### (II) Separation of the enriched t10, c12 CLA:

Residual lauryl alcohol was removed at 130°C at 25-35ml per minute by molecular distillation. The residue was coarsely separated into the lauryl esters (enriched in c9,t11 CLA) and free acids (enriched in t10,c12 CLA) by evaporation at 158°C at a flow rate of 25-35ml per minute.

### Isolation of the enriched t10, c12 CLA

The CLA free acids from step (II) above were distilled again at 160-165'C and 20-30 ml/min to reduce the ester content. Residual lauryl alcohol was reduced further by a distillation at 131°C and 25-30 ml/min flow rate. In order to remove any residual lauryl alcohol, free alcohols were esterified to the fatty acids present in the reaction mix, using SP392 *Mucor miehei* lipase (5%, batch lux 0110 ex Novo Nordisk). The enriched t10,c12 CLA containing fatty acids were separated from the lauryl esters using molecular distillation under vacuum at 155°C at 15-20ml per minute. The composition of the enriched t10,c12 CLA generated by this method is set out in table 3 below:

**TABLE 3**

| **Composition of typical preparation of enriched t10.c12 CLA fatty acids (wt%):** | **B** |
|---|---|
| c9,t11 | 8.3 |
| t10,c12 | 53.9 (**80.5% of total CLA**) |
| c9,c11 & c10,c12 | 2.9 |
| t9,t11t & t10,12t | 1.1 |
| Other CLA | 0.7 |
| **Total CLA** | **66.9** |
| 16:0 | 13.6 |
| 16:1 | - |
| 18:0 | 4.6 |
| 18:1 | 10.3 |
| 18:2 (non-CLA) | 3.1 |
| Other fatty acid | 1.5 |

### Example 2 - Preparation of t10, c12 CLA triglycerides

Enriched t10, c12 CLA (10g) prepared according to example 1 was mixed with 1.01g (10.1%) of glycerol (Pricerine 9083 glycerine CP from Ellis and Everards) and 0.5g (approximately 5%) of SP392 *Mucor Miehei* non-specific lipase (Mucor Meihei Ex Novo Nordisk Batch Lux 0110) was added.
The mixed materials were stirred under vacuum in a rotary-evaporator at 60°C with a slight nitrogen bleed.

After 96 hours the reaction was stopped by filtering the mixture through a thin layer of celite super-cel filter aid on a Buchner filter collecting the CLA triglyceride oil phase, the composition of which is set out in table 4 below:

**TABLE 4**

| **Fatty Acid composition of the triglycerides** | **Relative Percentage of Total fatty acid Lipid** |
|---|---|
| c9,t11 | 8.3 |
| t10,c12 | 54.8 (**81.7% of total CLA**) |
| c9,c11 & c10,c12 | 2.7 |
| t9,t11t & t10,12t | 1.3 |
| Other CLA | 0 |
| **Total CLA** | **67.1** |
| 16:0 | 13.5 |
| 16:1 | 0.1 |
| 18:0 | 4.9 |
| 18:1 | 10.3 |
| 18:2 (non-CLA) | 3.4 |
| Other fatty acid | 0.7 |

### Example 3

This example demonstrates how LRAT/ARAT inhibitors within the scope of the present invention may be identified using the in vitro Microsomal Assay of the esterification of retinol.

### Method of in vitro microsomal esterification of retinol:

Microsomes are obtained as described in: J.C. Saari and D.L. Bredberg, "CoA and Non-CoA Dependent Retinol Esterification in Retinal Pigment Epithelium" J. Biol. Chem. 23, 8084-90 (1988).

A solution containing 0.1M sodium phosphate pH 7 buffer, 5mM dithiothreitol, 2 mg/ml bovine serum albumin, 40 micromolar palmitoyl CoA, 40 micromolar dilauroyl phosphatidyl choline, 10 micromolar retinol and a test compound or solvent blank, was incubated for 1 hour at 37°C with a microsomal fraction isolated from bovine retinal pigment epithelial cells. After incubation, the reaction was quenched by addition of an equal volume of ethanol, and the retinyl esters formed (retinyl palmitate from the ARAT catalysed reaction, and retinyl laurate from the LRAT catalysed reaction) were extracted with hexane. The hexane layer was removed, evaporated under nitrogen, and the residue analysed by HPLC on a 3.9x300 mm C18 reversed phase column using a 80% methanol in tetrahydrofuran mobile phase and fluorescence detection (325 nm excitation, 480 nm emission) to quantitate the retinyl esters. The quantity of ester formed in the presence of the solvent blank was taken as 100%, and this was used to calculate the percent inhibition of ester formation for the compounds tested. As a control, an aliquot of microsomes was inactivated by boiling for 5 minutes, which resulted in at least 95% inhibition of ester formation.

The results that were obtained are summarised in Table 5.

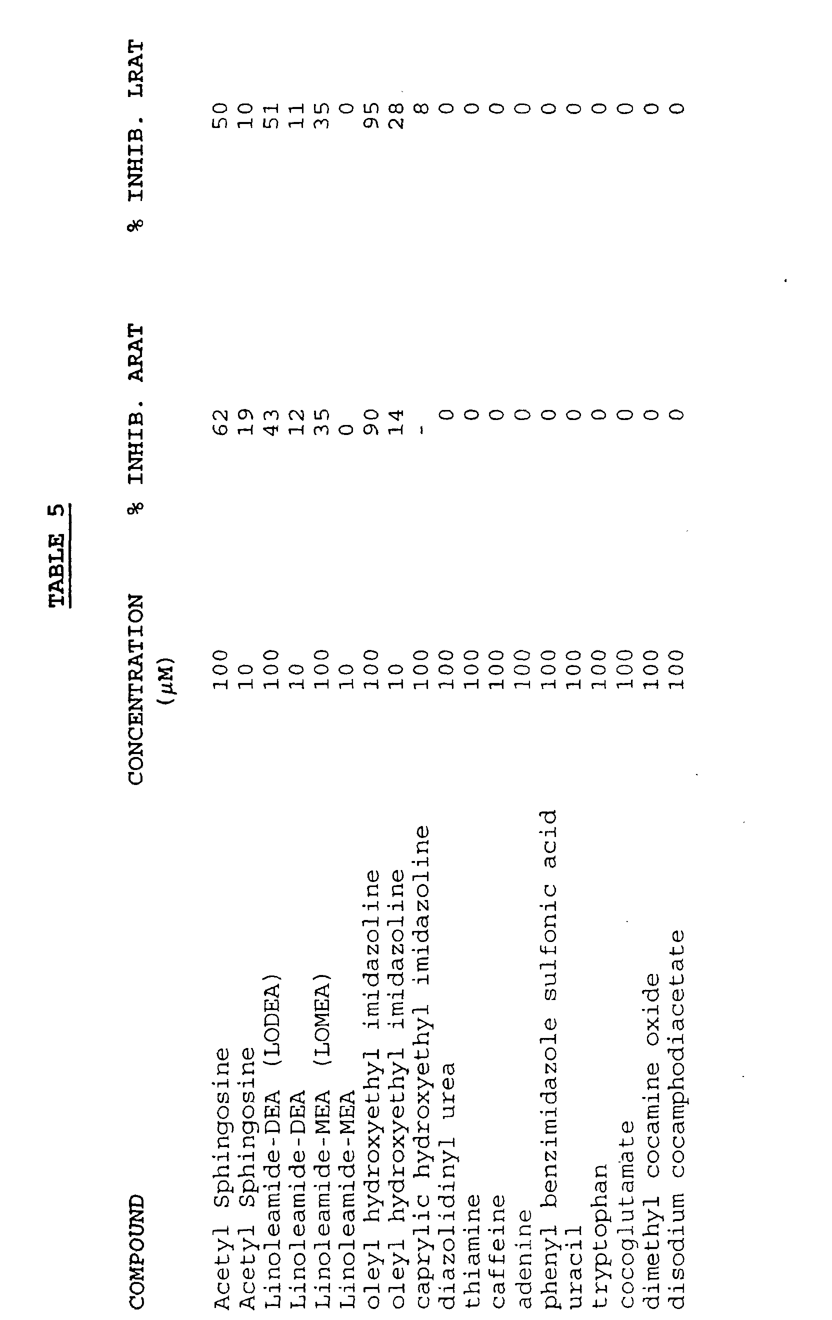

It can be seen that acetyl sphingosine, LODEA, LOMEA and hydroxyethyl imidazoline surfactant are potent retinol esterification inhibitors, while other surfactants and other heterocyclic compounds were essentially inactive. Caprylic hydroxyethyl imidazoline (R = CH₃(CH₂)₆) did not sufficiently inhibit LRAT.

The in vitro Microsomal Assay Test was run on the compounds listed in Tables 6A and 6B

The compounds in Table 6A were tested at a 100µM concentration. The compounds in Table 6B were tested at a 10µM concentration.

**TABLE 6A**

| **COMPOUND** | **% INHIBITION, ARAT** | **% INHIBITION, LRAT** |
|---|---|---|
| alpha damascone | 83 | 98 |
| Beta damascone | 84 | 92 |
| Delta damascone | 87 | 95 |
| Isodamascone | 80 | 92 |
| Damascenone | 70 | 79 |
| Alpha ionone | 45 | 49 |
| Beta ionone | 22 | 24 |
| Allyl alpha ionone | 22 | 36 |
| Isobutyl ionone | 8 | 45 |
| Alpha methyl ionone | 67 | 77 |
| Gamma methyl ionone | 21 | 38 |
| Brahmanol | 70 | 75 |
| Sandanol | 15 | 43 |
| Alpha terpineol | 26 | 25 |
| Timberol | 34 | 33 |
| Lyral | 76 | 71 |
| Tonalid | 50 | 33 |
| Ethyl saffranate | 51 | 49 |
| Traseolide | 41 | 21 |
| Sandalone | 23 | 12 |

**TABLE 6B**

| **COMPOUND** | **% INHIBITION, ARAT** | **% INHIBITION, LRAT** |
|---|---|---|
| Alpha damascone | 67 | 87 |
| Beta damascone | 45 | 52 |
| Delta damascone | 58 | 64 |
| Damascenone | 23 | 29 |
| Allyl alpha ionone | 16 | 17 |

It can be seen from the results in Tables 6A and 6B that certain cyclic aliphatic unsaturated compounds, in particular the ionones and damascones are potent inhibitors of LRAT and ARAT catalysed retinol esterification. These contain the trimethyl cyclohexene ring system present in retinol.

The in-vitro Microsomal Assay test was conducted with additional cyclic aliphatic unsaturated compounds. The results that were obtained are summarised in Table 7.

The compounds in Table 7 were tested at a 100 µM concentration.

**TABLE 7**

| **COMPOUND** | **% INHIBITION, ARAT** | **% INHIBITION, LRAT** |
|---|---|---|
| Dihydro alpha ionone | 13 | 18 |
| Alpha ionol | 0 | 0 |
| Beta ionol | 0 | 0 |
| Cinnamaldehyde | 0 | 0 |
| Vanillin | 0 | 0 |
| Eucalyptol | 0 | 0 |
| Menthol | 0 | 0 |
| Thymol | 0 | 0 |
| Carvone | 0 | 0 |
| Camphor | 0 | 0 |
| Mentone | 0 | 0 |
| Fenchyl alcohol | 12 | 4 |
| Isocyclogeraniol | 18 | 16 |
| Dimethyl ionone | 0 | 9 |
| Delta methyl ionone | 0 | 10 |

It can be seen from the results in Table 7 that not all cyclic aliphatic unsaturated compounds inhibit or sufficiently inhibit LRAT and ARAT catalysed retinol esterification.

The in-vitro Microsomal Assay test was conducted with a diterpene compound, geranyl geraniol or farnesol.

The results that were obtained are summarised in Table 8.

**TABLE 8**

| **COMPOUND** | **CONCENTRATION (µM )** | **% INHIB. ARAT** | **% INHIB. LRAT** |
|---|---|---|---|
| Geranyl Geraniol¹ | 100 | 81 | 77 |
| Geranyl Geraniol | 10 | 38 | 16 |
| Farnesol² | 100 | 43 | 43 |
| Farnesol | 10 | 20 | 10 |

| | | | |
|---|---|---|---|
| ¹ Obtained from TCI America (Portland, Oregon). Also available from Sigma and CTC Organics (Atlanta, Georgia). | | | |
| 2 Available from Givaudan Co., Bedoukian Co., or Dragoco Co. | | | |

It can be seen from the results in Table 8 that both geranyl geraniol and farnesol inhibit retinol esterification. Geranyl geraniol is a substantially more potent esterification inhibitor, than farnesol.

### Example 4

### Identification of Procollagen-I and Decorin Upregulation in Skin in Vivo Following Topical Retinoic Acid Treatment for Comparative Purposes

Collagen, the predominant matrix skin protein is known to impart tensile strength to skin. Decorin is a proteoglycan which is known to be important for controlled and correct deposition of collagen in the extracellular matrix of skin. It is also known in the art that the levels of collagen and decorin in skin are significantly reduced with aged and/or photodamaged skin. Many studies have shown that the levels of collagen type I in skin is decreased with age and/or with increased photodamage, (for example Lavker, R. J.Inv.Derm.,(1979),73,79-66; Griffiths et al. N. Eng. J. med. (1993) 329, 530-535). In the case of decorin, it has been shown that mRNA expression and expression of the proteoglycan is greatly reduced in photodamaged skin in vitro (Bernstein et al. Lab. Invest.(1995)72,662-669). The reduction of the levels of these skin proteins is accordingly associated with a decrease in the tensile strength of the skin causing wrinkles and laxity.

It is well known in the art that retinoic acid is a potent anti-aging active and induces dermal repair of photodamaged skin. It has been shown that wrinkle effacement and dermal repair following topical treatment of skin with retinoic acid arises through new collagen deposition and synthesis in the skin (for example, Griffiths et al. N. Eng. J. med. (1993) 329, 530-535). It is widely accepted that strengthening of the dermal matrix by boosting the level of collagen in skin using retinoic acid provides anti-ageing/dermal repair benefits. Procollagen I is a precursor of collagen. Increased production of procollagen I in response to a test compound application is a marker of an increased collagen level.

Two groups of women were recruited with identical or nearly identical degrees of mild to moderate photodamage on each outer forearm. They were supplied with 0.05% retinoic acid in a moisturising base (Retinova®) and also with a colour matched moisturising cream with similar sensory characteristics (Dermacare® lotion), but no active ingredients, as a placebo control. Each participant of the two groups applied the Retinova® to one outer forearm and placebo (Dermacare®) to the other outer forearm. Group 1 applied the products daily to their outer forearms for 14 weeks and the Group 2 applied the products to their outer forearms for 28 weeks. At the end of the studies two full thickness 4mm punch biopsies were taken from the treated areas of each forearm. Immunohistochemical analysis of the biopsy tissue taken from the participants was performed to identify the effect of retinoic acid treatment on the expression of the skin extracellular matrix components, decorin and procollagen-I, as compared with the placebo treated forearms. The following procedure was followed:

### MATERIALS

Antibody dilution buffer for wax sections was composed of Tris Buffered Saline (TBS), 3% bovine serum albumin (BSA), 0.05% Triton X-100 and 0.05% sodium azide. Primary antibodies for procollagen-I (amino terminal) were obtained from Chemicon International Inc. (cat# MAB 1912, rat IgGl) and used on wax sections at a dilution of 1:800, overnight at 4°C after the section had been pre-treated with trypsin (0.5 mg/ml, 25 minutes, 37°C). Primary antibodies for decorin were obtained from Biogenesis (rabbit polyclonal) and used on wax sections at a dilution of 1:800, overnight at 4°C. Anti-rat biotinylated secondary antibodies, obtained from DAKO (cat# E0468, rabbit polyclonal), were applied to wax sections at a dilution of 1:400. Anti-rabbit biotinylated secondary antibodies, obtained from Amersham (cat# RPN 1004, donkey polyclonal), were applied to wax sections at a dilution of 1:400. Streptavidin conjugated alkaline phosphatase, obtained from Zymed (cat# 43-4322), was used at a concentration of 1:2500. Fast Red chromogen was obtained from DAKO (cat# K597). Gills #3 Haemotoxylin nuclear counterstain obtained from Sigma (cat# GHS-3), was filtered and used without dilution. Trypsin was obtained from Sigma (cat# T-7186) and slides were mounted with Glycergel from DAKO (cat# C563).

### METHODS

Wax sections of the biopsy tissue were mounted on silane coated slides and baked for 18 hours at 55°C. The slides were dewaxed through xylene and alcohol and brought to water and then transferred to TBS. DAKO® pen was used to ring the sections. The sections were processed for antigen retrieval using trypsin where necessary, as indicated for each antibody. Where antigen retrieval was necessary, the slides were incubated for 25 minutes at 35°C with trypsin at 0.5 mg/ml (Sigma Cat # T-7186). The protease was subsequently rinsed off (2 x 2 minutes) with TBS. Following antigen retrieval, if necessary, or otherwise directly after ringing the sections, non specific antibody binding was blocked with 5% solutions of secondary antibody host serum in TBS/0.5% BSA/0.1% sodium azide as the blocking solution for at least 20 mins at room temperature in a humid chamber. The excess blocking solution was drained off, but the sections were not allowed to dry. The sections were then incubated with the primary antibody (appropriately diluted as indicated above) in a humid chamber overnight at 4°C. Antibody was subsequently drained from the sections, without allowing them to dry. The slides were then washed with TBS to remove unbound primary antibody - a one minute rinse followed by three five minute washes - and then incubated with the appropriate secondary antibody (appropriately diluted as indicated above) in a humid chamber for 1 hour at room temperature. The antibody solution was subsequently drained from the slides without allowing the section to dry. The slides were washed in TBS, a one minute rinse followed by 4 x 5 min washes, in order to remove the unbound secondary antibody. For the biotinylated secondary antibody the sections were subsequently incubated with streptavidin conjugate for 45 mins at 37°C and then washed in TBS to remove unbound streptavidin conjugate. The chromogen was added and the colour developed with observation to avoid over-staining. The sections were then counterstained and mounted.

Differences in the expression of procollagen-I and decorin between retionoic acid (Retinova®) and placebo (Dermacare®) treated sites were determined by visual assessment of the immunohistochemically stained sections using light microscopy.

This analysis identified marked upregulation of both procollagen-I and decorin in the photodamaged skin following topical application of retinoic acid (Retinova®), as set out in Table 9 below.

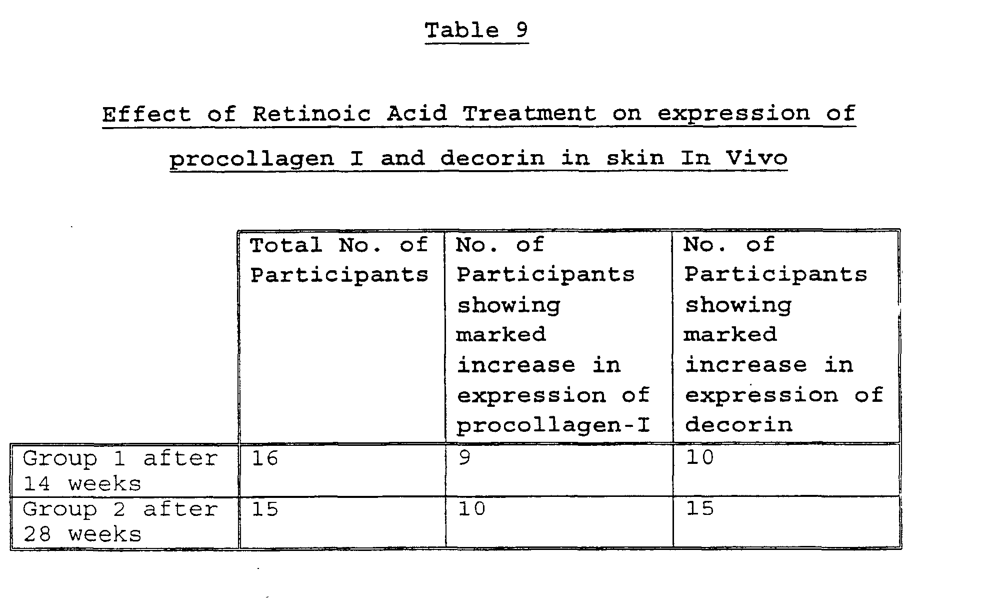

The extra cellular matrix components procollagen 1 and decorin are thus clearly identifiable markers of retinoic acid induced dermal repair.

### Example 5

### Procedure For Measuring Procollagen-I and Decorin Synthesis In Human Dermal Fibroblasts

### Preparation of Dermal Fibroblast Conditioned Medium

Primary human foreskin fibroblasts at passage 2 (P2) were seeded into 12-well plates at 10000 cells/cm² and maintained for 24 hours in an atmosphere of 5% carbon dioxide and 4% oxygen in Dulbeccos Modified Eagles Medium (DMEM) supplemented with 10% foetal calf serum. After this time the cells were washed with serum free DMEM and then incubated in fresh serum free DMEM for a further 60 hours. The fibroblast monolayers were then washed again with serum free DMEM. Test reagents and vehicle controls were added to the cells in triplicate in a final volume of 0.4ml/well fresh serum free DMEM and incubated for a further 24 hours. This fibroblast conditioned medium was either analysed immediately or snap frozen in liquid nitrogen and stored at -70°C for future analysis. The cells were then counted and data from the dot-blot analysis subsequently standardised to cell number.

### Example 6

### Dot Blot Assay for Procollagen-I and Decorin Protein in Dermal Fibroblast Conditioned Medium

Samples of conditioned medium from dermal fibroblasts treated with vehicle (as a control) or test reagents were supplemented with 20mM dithiothreitol (1:10 dilution of 200mM stock solution) and 0.1% sodium dodecylsulphate (1:100 dilution of 10% stock solution), mixed well and then incubated at 75°C for 2 minutes. A standard for the assay was generated by serial dilution of neat fibroblast conditioned medium from fibroblasts seeded at 10000 cells/cm² in a 175cm² flask and maintained in serum free DMEM as described above.

Assay samples were subsequently applied in triplicate to a prewetted sheet of Immobilon-P transfer membrane using the 96-well Bio-Dot Apparatus from Bio-Rad as described in the manufacturers guidelines. Approximately 200µl of medium was applied per well. The medium was allowed to filter through the membrane under gravity (30 minutes) after which the membrane was washed twice with PBS (200µl). These PBS washes were allowed to filter through the membrane under gravity (2x15 minutes). The Bio-Dot apparatus was then attached to a vacuum manifold and a third and final PBS wash carried out under suction. The apparatus was disassembled, the membrane removed and quickly cut as required before being placed in blocking buffer overnight at 4°C. Membranes prepared for decorin analysis were blocked with 3% (w/v) BSA/ 0.1% (v/v) Tween 20 in PBS, whilst those for procollagen-I analysis were blocked with 5% (w/v) non fat dried milk powder/ 0.05% Tween 20 in PBS.

The following day, the membranes were probed with 1:10000 dilution of primary antibodies to either human procollagen-I (MAB1912; rat monoclonal; Chemicon Int. Inc., Temecula, CA) or human decorin (rabbit polyclonal; Biogenesis) for 2 hours at room temperature. The membranes were subsequently washed with TBS/ 0.05% Tween 20 (3 x 5 minutes) and then incubated with 1:1000 dilution of ¹²⁵I-conjugated anti-rat or anti-rabbit F(ab')2 fragments (Amersham) as required for 1 hour at room temperature. Following this the Immobilon strips were again washed with TBS/Tween 20 (3 x 5 minutes) before being allowed to dry in air at room temperature. The dried membranes were wrapped in cellophane and exposed to a Molecular Dynamics storage phosphor screen for 16-18 hours.

At the end of this time the exposed screen was scanned by a phosphorimager (Molecular Dynamics Phosphorimager SF) using ImageQuant™ software. Dot intensity was assessed by computer-assisted image analysis using the quantification tools in ImageQuant™, standardised to cell number and the effects of various test reagents on decorin and procollagen-I synthesis were determined relative to a vehicle treated control value of 100 arbitrary units.

### Example 7

### TESTS

The table 10 below indicates the synergistic effect of conjugated linoleic acid in combination with the LRAT/ARAT inhibitor Ceramide 6 on decorin synthesis in human dermal fibroblasts, and the amounts in which the actives were applied. In order to normalise the results the effects of the test substances were determined relative to a vehicle treated control value of 100 arbitrary units. The concentrations of reagents used in the trials had no influence on cell viability.

**Table 10**

| **The Synergistic Effect on Decorin Synthesis by Conjugated Linoleic Acid in combination with an LRAT/ARAT inhibitor** | |
|---|---|
| **Treatment** | **Decorin** |
| Control (Vehicle) | 100 |
| 0.1 µM CLA | 96.8% |
| 0.01 µM Ceramide 6 | 101.2% |
| 0.1 µM CLA + 0.01 µM Ceramide 6 | 156.5% |

The results in table 10 indicate that the combination of conjugated linoleic acid with a LRAT/ARAT inhibitor significantly upregulates the synthesis of procollagen-I and/or decorin in human dermal fibroblasts as compared to the control.

The level of decorin in skin is associated with improved condition and appearance of skin. Increasing the level of decorin in skin is important for controlled and correct deposition of collagen in skin which is associated with many skin benefits such as wrinkle effacement and dermal repair of photodamaged skin.

### Synergistic effect of CLA with Retinoic Acid

The table 11 below indicates the synergistic effect of conjugated linoleic acid in combination with retinoic Acid, on procollagen-I synthesis in human dermal fibroblasts, and the amounts in which the actives were applied. In order to normalise the results the effects of the test substances were determined relative to a vehicle treated control value of 100 arbitrary units. The concentrations of reagents used in the trials had no influence on cell viability.

**Table 11**

| Untreated control = 100%. All results normalsed to this value. | |
|---|---|
| **Actives Tested** | **Procollagen 1** |
| 0.1 µM CLA | 78.9% |
| 0.01 µM trans Retinoic acid | 102.8% |
| 0.1 µM CLA + 0.01 µM trans RA | 124.2% |

The results in table 11 indicate that the combination of conjugated linoleic acid with a retinoic acid significantly upregulates the synthesis of procollagen-I in human dermal fibroblasts as compared to the control.

The level of decorin in skin is associated with improved condition and appearance of skin. Increasing the level of decorin in skin is important for controlled and correct deposition of collagen in skin, which is associated with many skin benefits such as wrinkle effacement and dermal repair of photodamaged skin.

### EXAMPLE 8

This example illustrates oil-in-water creams according to the invention.

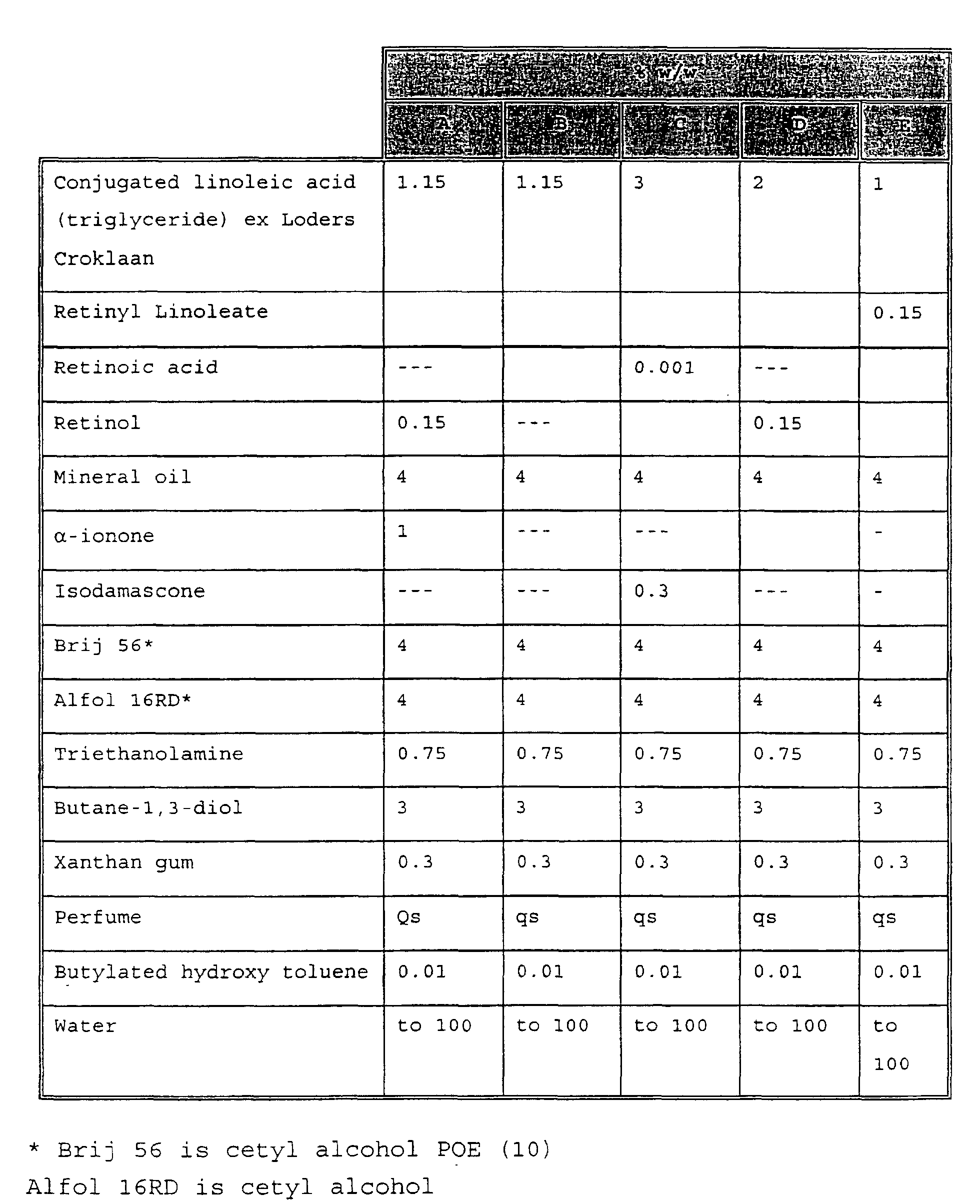

### EXAMPLE 9

This example illustrates alcoholic lotions according to the invention.

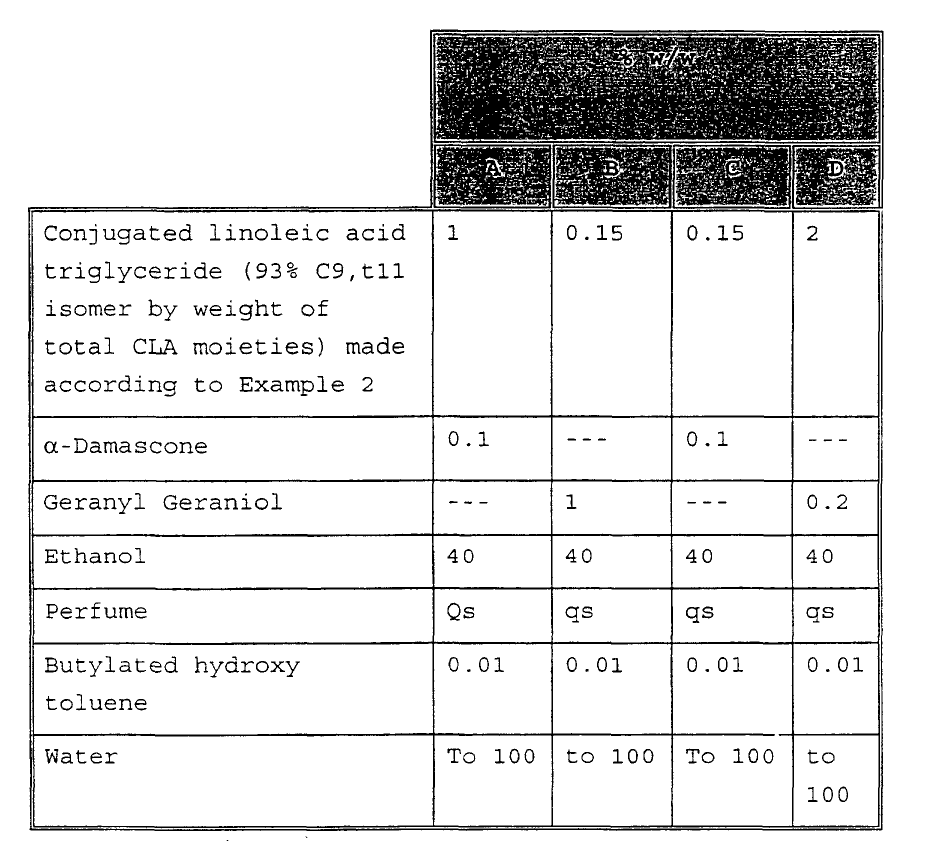

### EXAMPLE 10

This example illustrates a suncare cream incorporating the composition of the invention:

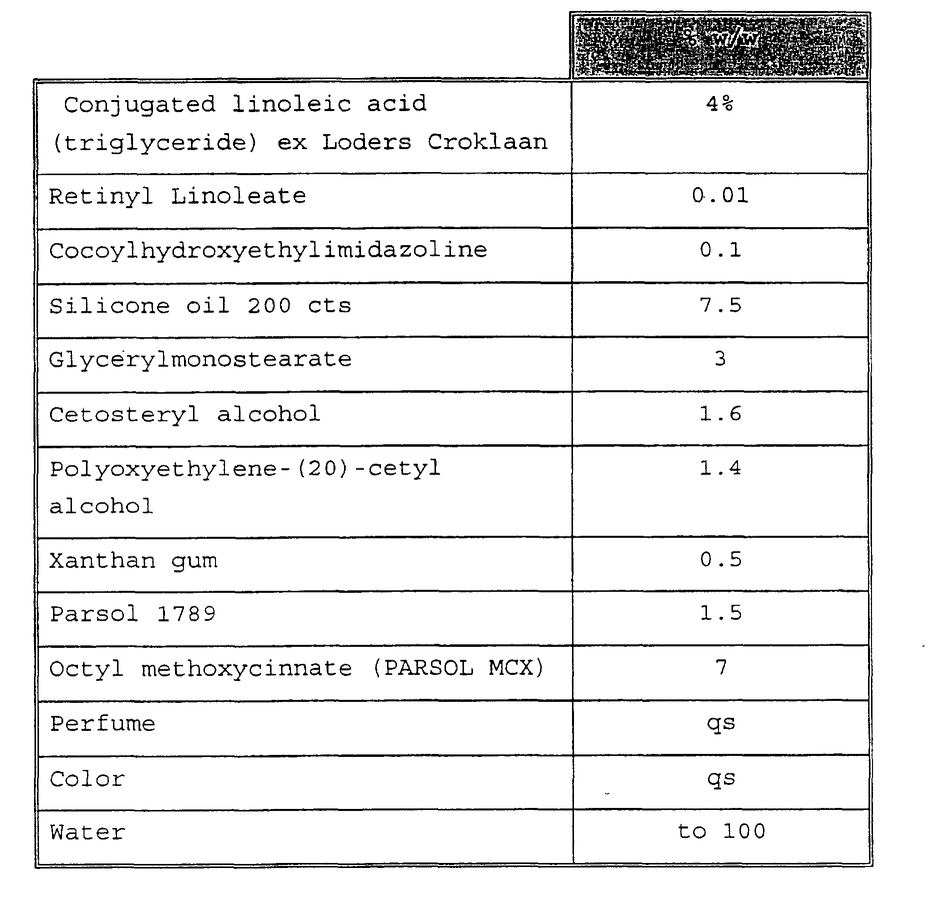

### EXAMPLE 11

This example illustrates a high internal phase water-in-oil emulsion incorporating the inventive composition.

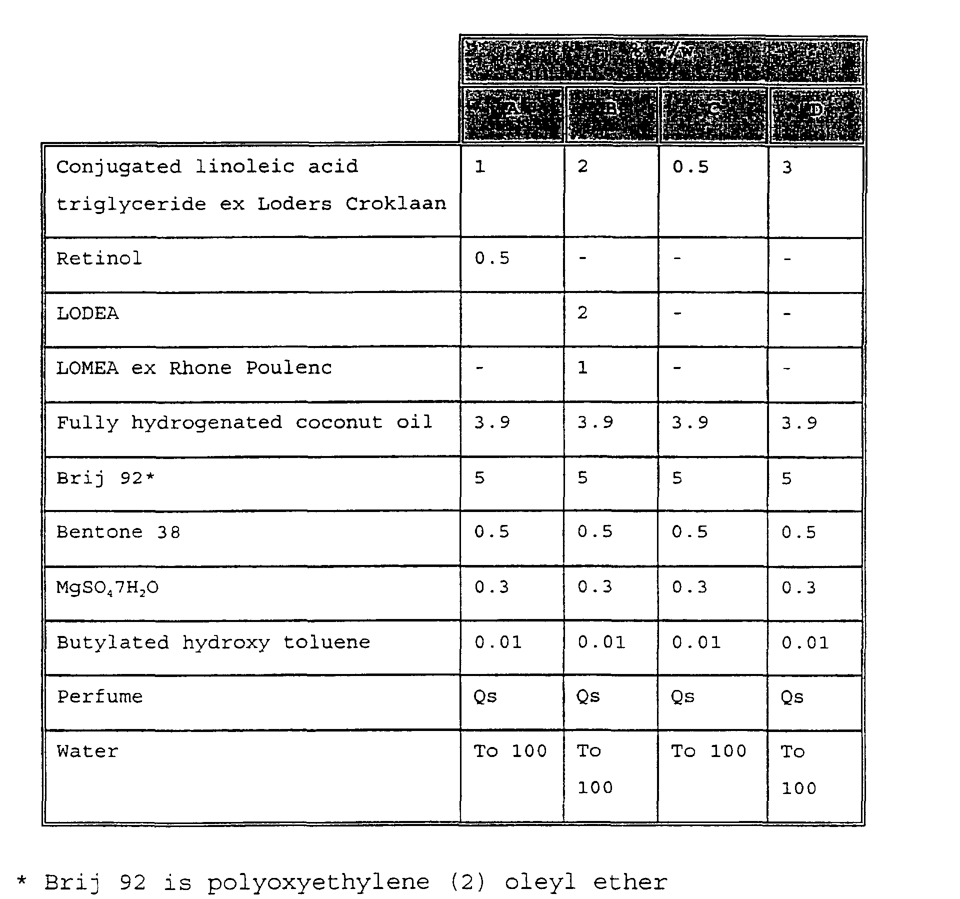

Examples 8 to 11 illustrate topical compositions according to the present invention. The compositions can be processed in conventional manner. They are suitable for cosmetic use. They provide an effective cosmetic treatment to improve the appearance of wrinkled, aged, photodamaged, and/or irritated skin, when applied to normal skin that has deteriorated through the aging or photoageing or when applied to youthful skin to help prevent or delay such deteriorative changes. The compositions are also effective for soothing irritated skin, conditioning dry skin, lightening skin colour and reducing oil and sebum secretions.

## Claims

1. A topical composition comprising:
(a) conjugated linoleic acid and/or a derivative thereof, wherein the linoleic acid derivative is a glyceride ester, an amide, a salt or a substituted conjugated linoleic acid;
(b) a retinoic acid, retinol, retinyl ester and/or a LRAT/ARAT inhibitor; and
(c) a dermatologically acceptable vehicle.

2. A topical composition according to claim 1, wherein the conjugated linoleic acid or derivatives thereof are the cis 9 trans 11 or the trans 10 cis 12 isomers.

3. A topical composition according to claim 1 or claim 2 wherein component (b) of the composition is a retinoic acid, retinol or a retinyl ester.

4. A topical composition according to claim 3, wherein component (b) is retinol or linoleamide monoethanolamide (MEA).

5. A topical composition according to any preceding claim wherein the retinyl ester is retinyl linoleate.

6. A topical composition according to any preceding claim, wherein the composition is a leave on composition.

7. A topical composition according to any of the preceding claims wherein at least 1% by weight of the conjugated linoleic moieties of the acid and/or derivatives thereof is present as the cis 9, trans 11 isomer and/or the trans 10, cis 12 isomer.

8. A topical composition according to any of the preceding claims, wherein the LRAT/ARAT inhibitor is a fatty amide, a hydroxy fatty acid amide, a ceramide, a melinamide, an imidazolidinones, a cyclic aliphatic unsaturated hydrocarbon, a terpene, or a fatty hydroxyethyl imadazoline surfactant, or mixtures thereof.

9. A topical composition according to claim 8, wherein the cyclic aliphatic unsaturated compound is selected from cyclic aliphatic unsaturated aldehydes, ketones, alcohols and esters such as alpha damascone, beta damascone, delta damascone, isodamascone, damascenone, alpha ionone, beta ionone, allyl alpha ionone, isobutyl ionone, alpha methyl ionone, gamma methyl ionone, brahmanol, sandanol, alpha terpineol, lyral, ethyl saffranate, and mixtures thereof.

10. A topical composition according to claim 9, wherein the cyclic aliphatic unsaturated compound is an α damascone or an α ionone.

11. A topical composition according to claim 8, wherein the fatty acid in the fatty acid amide is selected from linoleic acid, linolenic acid, arachidonic acid, gamma-linolenic acid, homo-gamma-linolenic acid, and mixtures thereof.

12. A topical composition according to claim 11, wherein the fatty acid in the fatty acid amide is linoleic acid.

13. A topical composition according to claim 8, wherein the hydroxy fatty acid amide is lactamide-monoethanolamide, C₁₃-β-hydroxy acid amide (2-hydroxy-C₁₃-amide), N-hydroxyethyl-2-hydroxy-C₁₆ amide, 12-hydroxy-N-(2-hydroxyethyl) octadecanamide, and monoethanolamide of castor oil, and mixtures thereof.

14. A topical composition according to claim 8, wherein the terpene is a pentacyclic triterpene monocarboxylic acid.

15. A topical composition according to claim 8, wherein the ceramide is a ceramide derivative which is acetyl sphingosine.

16. A cosmetic method of providing at least one skin care benefit selected from: treating/preventing wrinkling, sagging, dry, aged and/or photodamaged skin; boosting collagen levels in skin, boosting/maintaining decorin levels in skin, enhancing tissue repair; soothing irritated, red and/or sensitive skin; improving skin texture, smoothness and/or firmness; lightening skin; controlling oil/sebum secretion; the method comprising applying to the skin a topical composition as claimed in any preceding claim.

17. Cosmetic use of a composition as claimed in any of claims 1 to 15 for providing at least one skin care benefit selected from treating/preventing wrinkling, sagging, aged, dry, and/or photodamaged skin; boosting/maintaining collagen levels in skin, boosting/maintaining decorin levels in skin, enhancing tissue repair; soothing irritated, red and/or sensitive skin; improving skin texture, smoothness and/or firmness; lightening skin; controlling oil/sebum secretion.

18. Cosmetic use of conjugated linoleic acid and/or derivatives thereof in combination with a retinoic acid, retinol, retinyl ester and/or a LRAT/ARAT inhibitor in a cosmetic topical composition for providing at least one cosmetic skin care benefit selected from treating/preventing wrinkling, sagging, aged and/or photodamaged skin; boosting/maintaining collagen levels in skin, boosting/maintaining decorin levels in skin, enhancing tissue repair; soothing irritated, red and/or sensitive skin; improving skin texture, smoothness and/or firmness; lightening skin; controlling oil/sebum secretion.

## Patentansprüche

1. Topische Zusammensetzung, umfassend:
(a) konjugierte Linolsäure und/oder ein Derivat derselben, wobei das Linolsäure-Derivat ein Glyceridester, ein Amid, ein Salz oder eine substituierte konjugierte Linolsäure ist;
(b) eine Retinoesäure, ein Retinol, einen Retinylester und/oder einen LRAT/ARAT-Inhibitor; und
(c) ein dermatologisch annehmbares Vehikel.

2. Topische Zusammensetzung nach Anspruch 1, in der die konjugierte Linolsäure oder deren Derivate das cis-9-trans-11- oder das trans-10-cis-12-Isomer sind.

3. Topische Zusammensetzung nach Anspruch 1 oder Anspruch 2, in der die Komponente (b) der Zusammensetzung eine Retinoesäure, ein Retinol oder ein Retinylester ist.

4. Topische Zusammensetzung nach Anspruch 3, in der die Komponente (b) Retinol oder Linolamid-monoethanolamid (-MEA) ist.

5. Topische Zusammensetzung nach irgendeinem vorangehenden Anspruch, in der der Retinylester Retinyllinoleat ist.

6. Topische Zusammensetzung nach irgendeinem vorangehenden Anspruch, in der die Zusammensetzung eine Zusammensetzung zum Aufgetragenlassen ist.

7. Topische Zusammensetzung nach irgendeinem der vorangehenden Ansprüche, in der mindestens 1 Gewichts-% der konjugierten Linol-Einheiten der Säure und/oder Derivate derselben als das cis-9-trans-11-Isomer und/oder als das trans-10-cis-12-lsomer vorliegt.

8. Topische Zusammensetzung nach irgendeinem der vorangehenden Ansprüche, in der der LRAT/ARAT-Inhibitor ein Fettamid, ein Hydroxyfettsäureamid, ein Ceramid, ein Melinamid, ein Imidazolidinon, ein cyclischer aliphatischer ungesättigter Kohlenwasserstoff, ein Terpen oder ein Fetthydroxyethylimidazolin-Tensid oder deren Mischungen ist.

9. Topische Zusammensetzung nach Anspruch 8, in der die cyclische aliphatische ungesättigte Verbindung ausgewählt ist aus cyclischen aliphatischen ungesättigten Aldehyden, Ketonen, Alkoholen und Estern, wie alpha-Damascon, beta-Damascon, delta-Damascon, Isodamascon, Damascenon, alpha-lonon, beta-Ionon, Allyl-alpha-ionon, Isobutylionon, alpha-Methylionon, gamma-Methylionon, Brahmanol, Sandanol, alpha-Terpineol, Lyral, Ethylsafranat und deren Mischungen.

10. Topische Zusammensetzung nach Anspruch 9, in der die cyclische aliphatische ungesättigte Verbindung ein alpha-Damascon oder ein alpha-Ionon ist.

11. Topische Zusammensetzung nach Anspruch 8, in der die Fettsäure in dem Fettsäureamid aus Linolsäure, Linolensäure, Arachidonsäure, gamma-Linolensäure, Homo-gamma-linolensäure und deren Mischungen ausgewählt ist.

12. Topische Zusammensetzung nach Anspruch 11, in der die Fettsäure in dem Fettsäureamid Linolsäure ist.

13. Topische Zusammensetzung nach Anspruch 8, in der das Hydroxyfettsäureamid ein Lactamidmonoethanolamid, C₁₃-β-Hydroxysäureamid (2-Hydroxy-C₁₃-amid), N-Hydroxyethyl-2-hydroxy-C₁₆-amid, 12-Hydroxy-N-(2-hydroxyethyl)octadecanamid und Monoethanolamid von Rizinusöl und deren Mischungen ist.

14. Topische Zusammensetzung nach Anspruch 8, in der das Terpen eine pentacyclische Triterpenmonocarbonsäure ist.

15. Topische Zusammensetzung nach Anspruch 8, in der das Ceramid ein Ceramid-Derivat ist, bei dem es sich um Acetylsphingosin handelt.

16. Kosmetisches Verfahren zur Bereitstellung mindestens eines Hautpflege-Vorteils, der ausgewählt ist aus: Behandlung/Verhütung von faltiger, abgesackter, trockener, gealterter und/oder lichtgeschädigter Haut; Auffrischen des Kollagengehalts in der Haut, Auffrischen/Aufrechterhalten des Decorin-Gehalts in Haut, Verbesserung der Gewebereparatur; Beruhigung von gereizter, roter und/oder empfindlicher Haut; Verbesserung der Hautstruktur, -glätte und/oder -festigkeit; Aufhellen der Haut; Steuerung der Öl/Sebum-Sekretion; wobei das Verfahren umfasst, dass man auf der Haut eine topische Zusammensetzung nach irgendeinem vorangehenden Anspruch aufträgt.

17. Kosmetische Verwendung einer Zusammensetzung nach irgendeinem der Ansprüche 1 bis 15 zur Bereitstellung mindestens eines Hautpflege-Vorteils, der ausgewählt ist aus: Behandlung/Verhütung von faltiger, abgesackter, trockener, gealterter und/oder lichtgeschädigter Haut; Auffrischen des Kollagengehalts in der Haut, Auffrischen/Aufrecherhalten des Decorin-Gehalts in der Haut, Verbesserung der Gewebereparätur; Beruhigung von gereizter, roter und/oder empfindlicher Haut; Verbesserung der Hautstruktur, -glätte und/oder -festigkeit; Aufhellen der Haut; Steuerung der Öl/Sebum-Sekretion.

18. Kosmetische Verwendung einer von konjugierter Linolsäure und/oder Derivaten derselben in Kombination mit einer Retinoesäure, einem Retinol, einem Retinylester und/oder einem LRAT/ARAT-Inhibitor in einer kosmetischen topischen Zusammensetzung zur Bereitstellung mindestens eines kosmetischen Hautpflege-Vorteils, der ausgewählt ist aus Behandlung/Verhütung von faltiger, abgesackter, trockener, gealterter und/oder lichtgeschädigter Haut; Auffrischen des Kollagengehalts in der Haut, Auffrischen/Aufrechterhalten des Decorin-Gehalts in der Haut, Verbesserung der Gewebereparatur; Beruhigung von gereizter, roter und/oder empfindlicher Haut; Verbesserung der Hautstruktur, -glätte und/oder -festigkeit; Aufhellen der Haut; Steuerung der Öl/Sebum-Sekretion.

## Revendications

1. Composition topique comprenant :
(a) un acide linoléique conjugué et/ou un dérivé de celui-ci, dans lequel le dérivé d'acide linoléique est un ester de glycéride, une amide, un sel ou un acide linoléique substitué ;
(b) un acide rétinoïque, du rétinol, de l'ester de rétinyle et/ou un inhibiteur de LRAT/ARAT ; et
(c) un véhicule acceptable d'un point de vue dermatologique.

2. Composition topique selon la revendication 1, dans laquelle l'acide linoléique conjugué ou les dérivés de celui-ci sont les isomères cis 9 trans 11 ou trans 10 cis 12.

3. Composition topique selon la revendication 1 ou la revendication 2, dans laquelle le composant (b) de la composition est un acide rétinoïque, du rétinol ou un ester de rétinyle.

4. Composition topique selon la revendication 3, dans laquelle le composant (b) est du rétinol ou de la monoéthanolamide de linoléamide (MEA).

5. Composition topique selon l'une quelconque des revendications précédentes, dans laquelle l'ester de rétinyle est du linoléate de rétinyle.

6. Composition topique selon l'une quelconque des revendications précédentes, dans laquelle la composition est une composition destinée à être laissée en place.

7. Composition topique selon l'une quelconque des revendications précédentes, dans laquelle au moins 1 % en poids des parties de molécules linoléiques conjuguées de l'acide et/ou des dérivés de celui-ci, sont présentes sous la forme de l'isomère cis 9, trans 11 et/ou de l'isomère trans 10, cis 12.

8. Composition topique selon l'une quelconque des revendications précédentes, dans laquelle l'inhibiteur de LRAT/ARAT est une amide grasse, une amide d'acide hydroxy gras, une céramide, une mélinamide, une imidazolidinone, un hydrocarbure cyclique aliphatique insaturé, un terpène ou un tensioactif gras d'ahydroxyéthyle imidazoline, ou des mélanges de ceux-ci.

9. Composition topique selon la revendication 8, dans laquelle le composé insaturé cyclique aliphatique est sélectionné à partir des aldéhydes, des cétones, des alcools et des esters insaturés cycliques aliphatiques tels que l'alpha damascone, le bêta damascone, l'iso damascone, le damascénone, l'alpha ionone, le bêta ionone, l'allyle alpha ionone, l'isobutyle ionone, l'alpha méthyle ionone, le gamma méthyle ionone, le brahmanol, le sandanol, l'alpha terpinéol, le lyral, le saffranate d'éthyle et les mélanges de ceux-ci.

10. Composition topique selon la revendication 9, dans laquelle le composé insaturé cystique aliphatique est un α - damascone ou un α - ionone.

11. Composition topique selon la revendication 8, dans laquelle l'acide gras dans l'amide d'acide gras est sélectionné parmi l'acide linoléique, l'acide linolénique, l'acide arachidionique, l'acide gamma linolénique, l'acide homo gamma linolénique et les mélanges de ceux-ci.

12. Composition topique selon la revendication 11, dans laquelle l'acide gras dans l'amide d'acide gras est de l'acide linoléiquc.

13. Composition topique selon la revendication 8, dans laquelle l'amide d'acide hydroxy gras est de la monoéthanolamide de lactamide, de l'amide de C₁₃ - β - hydroxy acide (2 - hydroxy - C₁₃ - amide), de l'amide de N - hydroxy éthyle - 2 - hydroxy - C₁₆, de l'octadécanamide de 12 - hydroxy - N - (2 - hydroxy éthyle) et de la monoéthanolamide d'huile de ricin, et des mélanges de ceux-ci.

14. Composition topique selon la revendication 8, dans laquelle le terpène est un acide monocarboxylique de triterpène pentacyclique.

15. Composition selon la revendication 8, dans laquelle la céramide est un dérivé de céramide qui de l'acétyle sphingosine.

16. Procédé cosmétique pour fournir au moins un avantage de soin de la peau, sélectionné parmi : le traitement/la prévention des rides, de la desquamation, de la sécheresse, du vieillissement et/ou du photo endommagement de la peau ; l'augmentation des niveaux de collagène dans la peau, l'augmentation/le maintient des niveaux de décorine dans la peau, l'amélioration de la réparation des tissus ; l'apaisement de la peau irritée, rouge et/ou sensible ; l'amélioration de la texture, de la douceur et/ou de la fermeté de la peau ; l'éclaircissement de la peau ; le contrôle de la sécrétion d'huile/de sébum ; ledit procédé comprenant le fait d'appliquer sur la peau une composition topique selon l'une quelconque des revendications précédentes.

17. Utilisation cosmétique d'une composition selon l'une quelconque des revendications 1 à 15 afin de fournir au moins un avantage de soin de la peau, sélectionné parmi : le traitement/la prévention des rides, de la desquamation, de la sécheresse, du vieillissement et/ou du photo endommagement de la peau ; l'augmentation des niveaux de collagène dans la peau, l'augmentation/le maintient des niveaux de décorine dans la peau, l'amélioration de la réparation des tissus ; l'apaisement de la peau irritée, rouge et/ou sensible ; l'amélioration de la texture, de la douceur et/ou de la fermeté de la peau ; l'éclaircissement de la peau ; le contrôle de la sécrétion d'huile/de sébum.

18. Utilisation cosmétique d'acide linoléique et/ou de dérivés de celui-ci en combinaison avec un acide rétinoïque, du rétinol, un ester de rétinyle et/ou un-inhibiteur de LRAT/ARAT dans une composition cosmétique topique pour fournir au moins un avantage de soin de la peau, sélectionné parmi : le traitement/la prévention des rides, de la desquamation, de la sécheresse, du vieillissement et/ou du photo endommagement de la peau ; l'augmentation des niveaux de collagène dans la peau, l'augmentation/le maintient des niveaux de décorine dans la peau, l'amélioration de la réparation des tissus ; l'apaisement de la peau irritée, rouge et/ou sensible ; l'amélioration de la texture, de la douceur et/ou de la fermeté de la peau ; l'éclaircissement de la peau ; le contrôle de la sécrétion d'huile/de sébum.
